# EUROPEAN PATENT APPLICATION

(11) **EP 4 556 019 A1**
(43) Date of publication of application: **21.05.2025**
(21) Application number: 23854750.9
(22) Date of filing: 11.07.2023
(51) Int. Cl.: A61K 39/00, A61K 39/385, A61K 39/395, A61K 47/68, A61P 17/00, A61P 43/00, C07K 16/18

(54) **ANTIGEN COMPOSITION, COMPOSITION FOR ANTIGEN EXPRESSION, AND ANTIBODY COMPOSITION**

(30) Priority: 19.08.2022 JP 2022131234
(71) Applicant: Ushio Denki Kabushiki Kaisha, Tokyo 100-8150 (JP)
(72) Inventor: SHIMADA,Shun, Tokyo 100-8150 (JP)
(74) Representative: Maiwald GmbH
(86) International application number: PCT/JP2023/025593
(87) International publication number: WO 2024/038713

(57) **Abstract**

This invention provides method for inducing immunity targeting photo aged cells, or antibodies or binding fragments thereof targeting photo aged cells. Specifically, the invention provides antigen composition which contain at least one polypeptide selected from the group consisting of immunogenic GPR17 polypeptides, immunogenic CD34 polypeptides, immunogenic GABRR1 polypeptides, immunogenic OR2AG2 polypeptides, immunogenic CMKLR1 polypeptides, immunogenic CDH19 polypeptides, immunogenic CD93 polypeptides, immunogenic AVPR2 polypeptide, immunogenic CCR7 polypeptide, and immunogenic OXGR1 polypeptide.

Such at least one polypeptide is bound to a carrier protein or not bound to a carrier protein. The antigen composition is for inducing immunity against photo aged cells in a subject.

## Description

### TECHNICAL FIELD

The present invention relates to an antigen composition and a composition for antigen expression for inducing immunity against a photoaged cell, and an antibody composition having binding ability to a photoaged cell.

### BACKGROUND ART

Cellular aging is roughly classified into two types: photoaging due to ultraviolet irradiation and natural aging due to aging or the like, and has different characteristics. For example, photoaging of the skin frequently occurs in a part exposed to sunlight such as the face, neck, and back of the hand, and causes spots, wrinkles (particularly deep wrinkles), sagging, thickening of the skin, coloring of yellow or brown of the skin and the like. On the other hand, natural aging of the skin proceeds regardless of sunlight irradiation. In natural aging, spots are hardly formed, and fine and shallow wrinkles are formed. Also, in natural aging, the skin tends to shrink, and the skin is not colored.

However, senescence-associated acid β-galactosidase (SA-β-Gal), a cyclin-dependent kinase inhibitor (p16), and the like have been known as markers of natural aging of cells (Non-Patent Document 1), but cell markers specific to photoaging have not been known.

### PRIOR ART DOCUMENT

### NON-PATENT DOCUMENT

Non-Patent Document 1: Japanese journal of geriatrics, 2016, Vol. 53, pp. 88-94

### SUMMARY OF THE INVENTION

### PROBLEMS TO BE SOLVED BY THE INVENTION

In order to prevent or improve photoaging, a means for selectively exerting pharmacological activity and an immune response to a normal cell and a photoaged cell is required.

However, SA-β-Gal and a cyclin-dependent kinase inhibitor (p16), which are known cell aging markers, are not specific to a photoaged cell and are not present on the cell surface, and therefore cannot be used for drug therapy targeting a living photoaged cell.

Therefore, an object of the present invention is to provide a means for inducing immunity targeting a photoaged cell, or to provide an antibody or binding fragment thereof targeting a photoaged cell.

### MEANS FOR SOLVING THE PROBLEMS

As a result of intensive studies, the present inventors first searched for genes specifically expressed in a photoaged cell and not expressed in a naturally aged cell, and identified 10 genes of GPR17, CD34, GABRR1, OR2AG2, CMKLR1, CDH19, CD93, AVPR2, CCR7, and OXGR1 as photoaged cell surface markers.

Then, the present inventors completed the present invention based on the following findings.
(1) An antigen composition containing at least one polypeptide selected from the group consisting of GPR17, CD34, GABRR1, OR2AG2, CMKLR1, CDH19, CD93, AVPR2, CCR7, and OXGR1 proteins induces immunity against a photoaged cell.
(2) The antigen composition can be used for reducing or suppressing an increase in photoaged cells based on the induction of immunity.
(3) An antibody composition containing an antibody or binding fragment thereof having binding ability to at least one polypeptide selected from the group consisting of GPR17, CD34, GABRR1, OR2AG2, CMKLR1, CDH19, CD93, AVPR2, CCR7, and OXGR1 proteins can specifically target a photoaged cell.
(4) The antibody or binding fragment thereof targets a photoaged cell, so that the antibody composition can be used for specific delivery of a drug to a photoaged cell or for reducing or suppressing an increase in photoaged cells.

The present invention includes the following aspects.
[1] An antigen composition for inducing immunity against a photoaged cell in a subject, comprising
   at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide,
   in which the at least one polypeptide is bound to a carrier protein or is not bound to a carrier protein.
[2] An antigen composition for reducing or suppressing an increase in photoaged cells of a subject, comprising
   at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide,
   in which the at least one polypeptide is bound to a carrier protein or is not bound to a carrier protein.
[3] The antigen composition according to [1] or [2], comprising an immunogenic GPR17 polypeptide.
[4] The antigen composition according to any one of [1] to [3], in which the immunogenic GPR17 polypeptide includes 3 to 40 consecutive amino acid residues in a region selected from the group consisting of positions 1 to 51, positions 123 to 127, positions 198 to 212 and positions 293 to 295 of GPR17 long isoform.
[5] The antigen composition according to any one of [1] to [4], in which the immunogenic GPR17 polypeptide includes 3 to 40 consecutive amino acid residues in the region at positions 1 to 51 of the GPR17 long isoform.
[6] The antigen composition according to any one of [1] to [5], in which the immunogenic GPR17 polypeptide includes a polypeptide consisting of an amino acid sequence of SEQ ID NO: 5.
[7] The antigen composition according to any one of [1] to [6], in which the at least one polypeptide has 80% or more identity to a corresponding wild-type polypeptide.
[8] The antigen composition according to any one of [1] to [7], further comprising an adjuvant.
[9] The antigen composition according to any one of [1] to [8], for preventing or improving a photoaging state.
[10] A composition for antigen expression for stimulating immunity against a photoaged cell in a subject, comprising a polynucleotide encoding at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide.
[11] A composition for antigen expression for reducing or suppressing an increase in photoaged cells of a subject, comprising a polynucleotide encoding at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide.
[12] The composition for antigen expression according to [10] or [11], comprising a polynucleotide encoding an immunogenic GPR17 polypeptide.
[13] The composition for antigen expression according to any one of [10] to [12], in which the immunogenic GPR17 polypeptide includes 3 to 40 consecutive amino acid residues in a region selected from the group consisting of positions 1 to 51, positions 123 to 127, positions 198 to 212 and positions 293 to 295 of GPR17 long isoform.
[14] The composition for antigen expression according to any one of [10] to [13], in which the immunogenic GPR17 polypeptide includes 3 to 40 consecutive amino acid residues in the region at positions 1 to 51 of the GPR17 long isoform.
[15] The composition for antigen expression according to any one of [10] to [14], in which the immunogenic GPR17 polypeptide includes a polypeptide consisting of an amino acid sequence of SEQ ID NO: 5.
[16] The composition for antigen expression according to any one of [10] to [15], in which the at least one polypeptide has 80% or more identity to a corresponding wild-type polypeptide.
[17] The composition for antigen expression according to any one of [10] to [16], further comprising an adjuvant.
[18] The composition for antigen expression according to any one of [10] to [17], for preventing or improving a photoaging state.
[19] A method for inducing immunity against a photoaged cell, comprising administering to a subject:
   (i) at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide,
      the polypeptides being linked or not being linked to a carrier protein;
   (ii) a polynucleotide encoding at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide; or
   (iii) a combination of (i) and (ii).
[20] A method for reducing or suppressing an increase in photoaged cells of a subject, comprising administering to a subject in need thereof an effective amount of:
   (i) at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide,
      the polypeptides being linked or not being linked to a carrier protein;
   (ii) a polynucleotide encoding at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide; or
   (iii) a combination of (i) and (ii).
[21] An antibody composition for reducing or suppressing an increase in photoaged cells of a subject, comprising an antibody or antigen-binding fragment thereof having binding ability to at least one polypeptide selected from the group consisting of GPR17, CD34, GABRR1, OR2AG2, CMKLR1, CDH19, CD93, AVPR2, CCR7, and OXGR1 proteins.
[22] An antibody composition for delivering a drug to a photoaged cell of a subject, comprising an antibody or antigen-binding fragment thereof having binding ability to at least one polypeptide selected from the group consisting of GPR17, CD34, GABRR1, OR2AG2, CMKLR1, CDH19, CD93, AVPR2, CCR7, and OXGR1 proteins.
[23] The antibody composition according to [21] or [22], comprising an anti-GPR17 antibody or antigen-binding fragment thereof.
[24] A method for delivering a drug to a photoaged cell in a subject, comprising administering to a subject in need thereof:
   a composition containing an antibody or antigen-binding fragment thereof having binding ability to at least one polypeptide selected from the group consisting of GPR17, CD34, GABRR1, OR2AG2, CMKLR1, CDH19, CD93, AVPR2, CCR7, and OXGR1 proteins, and a drug.
[25] A method for reducing or suppressing an increase in photoaged cells of a subject, comprising administering to a subject in need thereof an effective amount of an antibody or antigen-binding fragment thereof having binding ability to at least one polypeptide selected from the group consisting of GPR17, CD34, GABRR1, OR2AG2, CMKLR1, CDH19, CD93,AVPR2, CCR7, and OXGR1 proteins.
[26] Use of:
   (i) at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide,
      the polypeptides being linked or not being linked to a carrier protein;
   (ii) a polynucleotide encoding at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide; or
   (iii) a combination of (i) and (ii),
   for the manufacture of a composition for (I) inducing immunity against a photoaged cell in a subject, (II) reducing or suppressing an increase in photoaged cells in a subject, or (III) preventing or improving a photoaging state in a subject.
[27] Use of:
   an antibody or antigen-binding fragment thereof having binding ability to at least one polypeptide selected from the group consisting of GPR17, CD34, GABRR1, OR2AG2, CMKLR1, CDH19, CD93, AVPR2, CCR7, and OXGR1 proteins for the manufacture of a composition for (A) delivering an agent to a photoaged cell in a subject, (B) reducing or suppressing an increase in photoaged cells in a subject, or (C) preventing or improving a photoaging state in a subject.

### EFFECT OF THE INVENTION

The antigen composition of the present invention contains a polypeptide derived from a cell surface marker of a photoaged cell, and can induce immunity against a photoaged cell in a subject by being administered to the subject.

The composition for antigen expression of the present invention comprises a polynucleotide encoding a polypeptide derived from a cell surface marker of a photoaged cell, and the polypeptide is expressed in a subject by administering the composition to the subject. As a result, the composition for antigen expression of the present invention can induce immunity against a photoaged cell.

The antibody composition of the present invention contains an antibody or binding fragment thereof having binding ability to a photoaged cell, and can target a photoaged cell.

### BRIEF DESCRIPTION OF THE DRAWINGS

Fig. 1 is a schematic diagram of the method for preparing a photoaged cell used in each Test Example.
Fig. 2 is microscopic images of normal human epidermal keratinocytes (NHEK) that have been irradiated with ultraviolet rays at various irradiation amounts obtained in Test Example 1.
Fig. 3 is a graph comparing mRNA amounts of various genes of NHEK without ultraviolet irradiation treatment and NHEK subjected to each treatment of 50 day subculture treatment (P50) and 80 mJ/cm² ultraviolet irradiation treatment, obtained by RNA-Seq analysis of Test Example 2. The vertical axis represents a normalized read count.
Fig. 4 is Hoechst stained images (Hoechst) and anti-GPR17 monoclonal antibody stained images (anti-GPR17) of NHEK (UV(+)) subjected to ultraviolet irradiation treatment at 80 mJ/cm² or NHEK (UV(-)) without ultraviolet irradiation treatment in Test Example 4, and bright field images (Bright field) of the same cells.

### MODE FOR CARRYING OUT THE INVENTION

In the present specification, the "photoaged cell" generally refers to a living cell that has become aged by ultraviolet irradiation, and has not become cancerous. The photoaged cell is not particularly limited, but has, for example, at least one of the characteristics (1) to (3) below, and preferably has all characteristics of (1) to (3) below:
(1) being β-galactosidase (SA-β-Gal) or cyclin-dependent kinase inhibitor (p16) positive;
(2) stop or significant reduction of cell division is observed; and
(3) the cell is enlarged.

Examples of the origin of the photoaged cell include a cell of the skin. Specific examples of the cell of the skin include epidermal cells such as a keratinocyte, a pigmented cell (melanocyte), a Langerhans cell, and a Merkel cell; dermal cells such as a fibroblast; adipose tissue cells such as an adipocyte; and hair follicle cells such as a hair papilla cell, a hair matrix cell, an inner root sheath cell, and an outer hair root sheath cell. Among them, as the origin of the photoaged cell, epidermal cells or dermal cells are preferable, a keratinocyte or a fibroblast is more preferable, and a keratinocyte is still more preferable.

In the present specification, the term "induce immunity" or "immune induction" against a specific substance (antigen) refers to causing, enhancing, accelerating, or prolonging a detectable immune response to the specific substance (antigen). Examples of the immune response include humoral immune system responses (for example, a response by a B cell, more specifically production and secretion of an antibody against a specific substance); cellular immune system responses (for example, activation of a T cell, an NK cell, more specifically activation of a T cell or NK cell against a specific substance); innate immune system responses (for example, activation of a Toll-like receptor signal system, secretion of lymphokines (cytokines or chemokines such as Th1, Th2, Th17), activation of a macrophage, activation of a dendritic cell, etc.), and proliferation promotion of an immune system cell (for example, proliferation promotion of a B cell that produces the antibody against a specific substance or a T cell or NK cell against a specific substance).

In the present specification, the "immunogenic" refers to the ability of a substance to induce immunity against the substance in a subject when the substance is administered to the subject, with binding ability to a carrier protein and/or combination of an adjuvant as necessary.

In the present specification, the "subject" or "administration subject" refers to an individual organism, and includes a patient having a disease or the like. The subject includes, for example, a human or a non-human animal (for example, a non-human mammal).

In the present specification, the "improvement" of a disease, a symptom or a health condition refers to recovering or alleviating a disease, a symptom or a health condition; preventing or delaying deterioration of a disease, a symptom or a health condition; or reversing, preventing, or delaying progression of a disease or a condition.

In the present specification, the "prevention" of a disease, a symptom or a health condition refers to delaying or preventing occurrence of a disease, a symptom or a health condition; reducing risk of occurrence of a disease, a symptom or a health condition; or reducing incidence of a disease, a symptom or a health condition.

In the present specification, the "photoaging state" refers to a disease, a condition or a health condition (including a condition that does not require medical treatment, such as an aesthetic change) caused by photoaging in a subject. The photoaging state includes, for example, a disease, a symptom or a health condition caused by an increase in photoaged cells.

GPR17 (G protein-coupled receptor 17) belongs to the G-protein-coupled receptor (GPCR) superfamily, and is a gene encoding a cysteinyl leukotriene receptor. GPR17 has two isoforms of a long isoform (367 amino acid residues in humans, NCBI RefSeq ID NP_001154887.1) and a short isoform that have N-terminal portions having different lengths due to a difference in splicing (339 amino acid residues in humans, NCBI RefSeq ID NP_001154888.1).

The Gene ID of NCBI (URL: https://www.ncbi.nlm.nih.gov/) of human GPR17 is 2840. The human GPR17 long isoform is obtained by adding a region of 28 residues to the N-terminus of the short isoform. Long isoform mRNA is expressed in heart and kidney, and short isoform mRNA is mainly expressed in brain.

In the present specification, the amino acid residue numbers of GPR17 polypeptides are represented by the amino acid residue numbers of human GPR17 when aligned with the human GPR17 long isoform.

CD34 is a gene encoding a transmembrane phosphorylated glycoprotein. For example, the NCBI Gene ID of human CD34 is 947.

GABRR1 (gamma-aminobutyric acid type A receptor subunit rho1) is a gene encoding a γ-aminobutyric acid (GABA) receptor, which is a ligand-dependent chloride channel. For example, the NCBI Gene ID of human CD34 is 2569.

OR2AG2 (olfactory receptor family 2 subfamily AG member 2) is a gene that belongs to the GPCR superfamily and encodes an olfactory receptor. For example, the NCBI Gene ID of human OR2AG2 is 338755.

CMKLR1 (chemerin chemokine-like receptor 1) is a gene encoding a GPCR whose ligand is chemerin, a chemotactic adipokine. For example, the NCBI Gene ID of human CMKLR1 is 1240.

CDH19 (cadherin 19) is a gene encoding cadherin 19, which is one of cadherins. For example, the NCBI Gene ID of human CDH19 is 28513.

CD93 is a gene encoding a C-type lectin transmembrane receptor. For example, the NCBI Gene ID of human CD93 is 22918.

AVPR2 (arginine vasopressin receptor 2) is a gene encoding vasopressin receptor type 2 belonging to the GPCR superfamily. For example, the NCBI Gene ID of human AVPR2 is 554.

CCR7 (C-C motif chemokine receptor 7) is a gene encoding a GPCR whose ligands are CCL19 and CCL21, which are chemokines. For example, the NCBI Gene ID of human CCR7 is 1236.

OXGR1 (oxoglutarate receptor 1) is a gene encoding a GPCR belonging to the oxoglutarate receptor family within the GPCR superfamily. For example, the NCBI Gene ID of human OXGR1 is 27199.

### [Antigen composition/composition for antigen expression]

### (Antigen composition, antigen polypeptide)

The antigen composition of the present invention contains a single polypeptide or a combination of two or more polypeptides selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide. In the present specification, these polypeptides may be collectively referred to as "antigen polypeptides".

In the present specification, the immunogenic GPR17 polypeptide refers to a polypeptide that is immunogenic to a native GPR17 protein of a subject (for example, a human native GPR17 protein) or to a cell expressing the native GPR17 protein. The immunogenic GPR17 polypeptide may be, for example, a polypeptide including or consisting of a full-length GPR17 long isoform or a full-length GPR17 short isoform of a subject or a portion thereof. Among them, the immunogenic GPR17 polypeptide is preferably a polypeptide including an extracellularly exposed region in the GPR17 protein of the subject.

In the present specification, the immunogenic CD34 polypeptide, the immunogenic GABRR1 polypeptide, the immunogenic OR2AG2 polypeptide, the immunogenic CMKLR1 polypeptide, the immunogenic CDH19 polypeptide, the immunogenic CD93 polypeptide, the immunogenic AVPR2 polypeptide, the immunogenic CCR7 polypeptide, and the immunogenic OXGR1 polypeptide refer to polypeptides that are immunogenic to a native protein encoded by the respective gene to be administered (for example, a human corresponding native protein, etc.), or to a cell expressing the native protein. These immunogenic polypeptides can each independently be, for example, polypeptides including or consisting of a full-length polypeptide encoded by a corresponding gene or a portion thereof. Among them, these immunogenic polypeptides are preferably polypeptides including an extracellularly exposed region in the protein encoded by the corresponding gene of the subject.

The antigen polypeptide can induce stronger immunity against a photoaged cell than a normal cell of the same type as the photoaged cell. Further, it is preferable that the antigen peptide does not induce immunity against a cell that is the same type as the photoaged cell and is not photoaged.

The number of amino acid residues of the antigen polypeptide is each independently, for example, 3 or more. From the viewpoint of more remarkably exhibiting the effect of the present invention, the number of amino acid residues can be preferably 5 or more, more preferably 8 or more, and still more preferably 10 or more, and can be 200 or less, 100 or less, 50 or less, 40 or less, 30 or less, or 20 or less.

The antigen polypeptide has an amino acid sequence identity of preferably 80% or more, more preferably 90% or more, still more preferably 95% or more, still more preferably 98% or more, and particularly preferably 99% or more with respect to the amino acid sequence identity to a corresponding wild-type polypeptide, from the viewpoint of more remarkably exhibiting the effect of the present invention.

In the present specification, the amino acid sequence identity is an identity value obtained by alignment with default parameters (Matrix=BLOSUM62; Gap Costs=Existence 11, Extension 1; Composition adjustment=No adjustment) in blastp of NCBI BLAST (https://blast.ncbi.nim.nih.gov/).

The antigen composition of the present invention preferably contains at least an immunogenic GPR17 polypeptide.

The immunogenic GPR17 polypeptide preferably includes or consists of 3 or more, 5 or more, 8 or more, or 10 or more consecutive amino acid residues in any region selected from the group consisting of positions 1 to 51, positions 123 to 127, positions 198 to 212 and positions 293 to 295 of the GPR17 long isoform as the extracellularly exposed region. Among them, it is more preferable that the polypeptide includes or consists of 3 to 40, 5 to 40, 8 to 40 or 10 to 40 consecutive amino acid residues in the region at positions 1 to 51 of the GPR17 long isoform.

Here, for example, the polypeptide has amino acid sequences represented by SEQ ID NO: 1 at positions 1 to 51, SEQ ID NO: 2 at positions 123 to 127, SEQ ID NO: 3 at positions 198 to 212, and SEQ ID NO: 4 (His-Gly-Ala) at positions 293 to 295 of the human GPR17 long isoform.

From the viewpoint of suppressing immune induction against the GPR17 short isoform, the immunogenic GPR17 polypeptide preferably includes or consists of, for example, amino acid residues at positions 1 to 36 of the human GPR17 long isoform represented by SEQ ID NO: 5. More preferably, the immunogenic GPR17 polypeptide includes or consists of 3 or more, 5 or more, 8 or more, or 10 or more consecutive amino acid residues in the region at positions 1 to 28 of the human GPR17 long isoform.
(1) SEQ ID NO: 1: Positions 1 to 51 of human GPR17 long isoform; amino acid sequence MSKRSWWAGS RKPPREMLKL SGSDSSQSMN GLEVAPPGLI TNFSLATAEQ C
(2) SEQ ID NO: 2: Positions 123 to 127 of human GPR17 long isoform; amino acid sequence NHWPF
(3) SEQ ID NO: 3: Positions 198 to 212 of human GPR17 long isoform; amino acid sequence PQTVQTNHTV VCLQL
(4) SEQ ID NO: 4: Positions 293 to 295 of human GPR17 long isoform; amino acid sequence HGA
(5) SEQ ID NO: 5: Positions 1 to 36 of human GPR17 long isoform; amino acid sequence MSKRSWWAGS RKPPREMLKL SGSDSSQSMN GLEVAP

The preparation method of the antigen polypeptide can be appropriately selected according to the number of amino acid residues, and for example, the antigen polypeptide may be chemically synthesized by a solid phase synthesis method or the like, or may be biosynthesized by translating mRNA encoding the polypeptide in a cell or a cell-free translation system. The obtained polypeptide can be appropriately purified by a known purification method such as chromatography and used.

Each of the antigen polypeptides may be post-translationally modified, or may be an unmodified polypeptide.

From the viewpoint of more remarkably exhibiting the effect of the present invention, the antigen polypeptide may be optionally bound to a carrier protein used for promoting immune induction. Here, the binding to the carrier protein is performed by covalent or non-covalent linking by chemical treatment, for example, in the presence of the antigen polypeptide, the carrier protein, and optionally a chemical substance such as a crosslinking agent. Alternatively, the binding to the carrier protein may be performed by translating the antigen polypeptide and the carrier protein as an integral polypeptide. Examples of the carrier protein include keyhole limpet hemocyanin (KLH), bovine serum albumin (BSA), chicken gamma globulin (CGG), and ovalbumin.

The antigen polypeptide may have an additional amino acid residue or an amino acid residue mutation to enhance binding ability to a major histocompatibility complex (MHC) or to facilitate peptide delivery intracellularly or to a specific tissue.

### (Composition for antigen expression, polynucleotide for antigen expression)

The composition for antigen expression of the present invention contains one or two or more polynucleotides encoding at least one of the antigen polypeptides (referred to herein as "polynucleotide for antigen expression"). When one polynucleotide encodes a plurality of polypeptides, two or more polypeptides may be configured to be expressed in a polycistronic fashion.

The polynucleotide for antigen expression is not particularly limited as long as it can express the antigen polypeptide in a subject. The polynucleotide for antigen expression is, for example, DNA or RNA, and may partially or entirely contain nucleotide modifications (for example, modifications related to improvement of nucleotide stability and translation efficiency, such as phosphorothioate (PS) modification and pseudouridine modification). Among them, the polynucleotide for antigen expression is preferably DNA or mRNA, and more preferably DNA from the viewpoint of storage stability.

The polynucleotide for antigen expression is preferably operably linked to at least one type of control element necessary for expression (for example, promoter, enhancer, terminator, silencer, polycistronic expression factor (IRES, 2A peptide sequence, etc.)), and more preferably operably linked to at least one promoter.

In the present specification, a component is "operably linked" refers to being positioned to allow the component to function in its intended manner. For example, a control element is operably linked to a gene refers that the control element is ligated to a polynucleotide encoding the gene so that the expression of the gene can be controlled in the intended manner of the control element.

In one embodiment, the polynucleotide for antigen expression can be an expression vector. The expression vector may contain at least one type of the control element, and more preferably contains at least one promoter. Also, the expression vector may further contain a secretory signal sequence, a tag sequence, an origin of replication, a polyA sequence, a multiple cloning site, a sorting marker, and the like.

The expression vector may be, for example, a plasmid vector, a cosmid vector, or a viral vector.

Examples of the viral vector include viral vectors such as adenovirus, adeno-associated virus, herpes simplex virus, cytomegalovirus, vaccinia virus, Epstein-Barr virus, and retrovirus (lentivirus, etc.).

These polynucleotides for antigen expression can be designed and prepared by various known genetic engineering techniques.

### (Form of composition)

A specific embodiment of each of the antigen composition and the composition for antigen expression is not particularly limited, includes, for example, a pharmaceutical composition (including a pharmaceutical product or a quasi-pharmaceutical product), a cosmetic composition, a food (including a functional food such as a food for specific health use, a food labeled with functionality, a nutritive functional food or a supplement), or feed, and is preferably a pharmaceutical composition or a cosmetic composition.

The dosage form of each of the antigen composition and the composition for antigen expression may be appropriately selected depending on the intended use, and may be, for example, a dry product (lyophilized product, powder, etc.), a solution, a dispersion, a suspension, a tablet, a capsule, a troche, a liposome, a paste, a mousse, a gel, an emulsion, a cream, an aerosol, a spray, a sheet (carrying the substrate), or the like.

The composition for antigen expression is preferably included in a vehicle (for example, viral particle, liposome, etc.) for delivering the polynucleotide for antigen expression into a cell.

### (Other components of composition)

Each of the antigen composition and the composition for antigen expression may optionally contain one or two or more additional components such as a carrier, an excipient, and a stabilizer depending on its use.

Each of the antigen composition and the composition for antigen expression may further contain at least one adjuvant that can be used for enhancing, accelerating, or prolonging immune induction from the viewpoint of more remarkably exhibiting the effect of the present invention. Examples of the adjuvant include at least one selected from minerals (aluminum hydroxide, aluminum phosphate, aluminum chloride, alum, etc.), toxins (CTB, E. coli heat-labile toxin, etc.), oil-in-water emulsions (MF59, AS03, etc.), water-in-oil emulsions (ISA51, ISA720, etc.), biopolymers (inulin polymer (Advax, etc.), heme polymer (Hemozoin, etc.), cyclodextrin, etc.), saponins (QS21, etc.), AS04, RC-529, AS02, AS01, flagellins, double-stranded RNA, Freund's incomplete adjuvants, Freund's complete adjuvants, STING ligands, CpG oligodeoxynucleotides, cytokines (IL-12, GM-CSF, etc.), DOTAP, DDA, and the like.

When the antigen composition or the composition for antigen expression is a pharmaceutical composition, for example, pharmaceutically acceptable water; salt solutions; phosphate buffered saline (PBS); pH buffer components (phosphoric acid, citric acid, boric acid buffer, sodium bicarbonate, Tris-CI, etc.); carbohydrates such as monosaccharides, disaccharides, oligosaccharides, and polysaccharides (dextrin, dextran, cellulose, chitin, chitosan, etc.); sugar alcohols such as mannitol, sorbitol, and maltitol; alcohols such as glycerol and ethanol; hydrophilic polymers such as polyvinylpyrrolidone and polyethylene glycol; amino acids such as glycine, glutamine, asparagine, histidine, lysine, and arginine; proteins such as serum albumin and gelatin; low-molecular polypeptides; preservatives such as benzalkonium chloride, benzethonium chloride, benzoic acid, salicylic acid, thimerosal, methylparaben, propylparaben, and phenethyl alcohol; and the like may be contained singly or in combination of two or more.

When the antigen composition or the composition for antigen expression is a cosmetic composition, for example, in addition to the components exemplified in the pharmaceutical composition, a moisturizer, an oily component, a surfactant, a colorant, an oxidation inhibitor, a sequestering agent, a refreshing agent, a fragrance, an ultraviolet absorbing/scattering agent, an antioxidant, a medicinal ingredient, and the like, which are acceptable for the cosmetic composition, may be contained singly or in combination of two or more.

### (Function and use)

As shown in Examples herein, the respective genes of GPR17, CD34, GABRR1, OR2AG2, CMKLR1, CDH19, CD93, AVPR2, CCR7, and OXGR1 have increased expression levels in a photoaged cell as compared with a corresponding normal cell. Therefore, the protein encoded by these genes can function as a photoaged cell-specific marker.

The antigen composition contains the full length or part of these markers. Therefore, when the antigen composition is administered to a subject, immunity against these proteins is induced, and as a result, immunity against a photoaged cell can be induced. Then, the antigen composition can reduce or suppress an increase in photoaged cells in the subject by the induced immunity, and can further prevent or improve a photoaging state.

When the composition for antigen expression is administered to a subject, the full length or part of the photoaged cell-specific marker encoded by the polynucleotide contained therein is expressed. As a result, similarly to the antigen composition, the composition for antigen expression can induce immunity against a photoaged cell in a subject. Then, the composition for antigen expression can reduce or suppress an increase in photoaged cells in the subject by the induced immunity, and can further prevent or improve a photoaging state.

According to the above, each of the antigen composition or the composition for antigen expression can be administered to a subject and used for inducing immunity against a photoaged cell in the subject.

In addition, each of the antigen composition or the composition for antigen expression can be administered to a subject and used for reducing or suppressing an increase in photoaged cells in the subject.

Furthermore, each of the antigen composition or the composition for antigen expression can be administered to a subject and used for preventing (so-called vaccine-like use) or improving a photoaging state in the subject.

Similarly, the antigen polypeptide, the polynucleotide for antigen expression, or a combination thereof can be used for producing a composition for (I) inducing immunity against a photoaged cell in a subject, (II) reducing or suppressing an increase in photoaged cells in a subject, or (III) preventing or improving a photoaging state in a subject.

Furthermore, the antigen polypeptide, the polynucleotide for antigen expression, or a combination thereof can be administered to a subject and used in a method for (I) inducing immunity against a photoaged cell in a subject, (II) reducing or suppressing an increase in photoaged cells in a subject by administering an effective amount thereof, or (III) preventing or improving a photoaging state in a subject.

The immune induction is preferably promotion of production of an antibody against a protein corresponding to the immunogenic polypeptide or promotion of cellular immunity against the protein, and more preferably the promotion of production of an antibody against a protein.

The prevention or improvement of a photoaging state may be medical treatment or non-therapeutic.

### (Subject)

The subject to be administered is, for example, a mammal, includes a human or a non-human mammal (a non-human primate, a dog, a cat, a cow, a horse, a sheep, a pig, etc.), and is preferably a human.

The subject may be, for example, a person in need of prevention or improvement of a photoaging state, for example, a person having a photoaging state or a person having a risk of a photoaging state.

### (Administration mode/dose)

Examples of the route of administration include percutaneous, intradermal, subcutaneous, transmucosal (nasal, sublingual, oral), intravascular (intravenous, etc.), intraperitoneal, intrathecal, intramuscular, and oral.

In the administration, the dose per administration can be appropriately set according to the type of the composition to be used, the administration route, the attribute of the subject, and the like, and can be, for example, 0.001 to 100 mg or 0.01 to 10 mg in terms of the amount of the antigen polypeptide per 1 kg of the body weight of the subject. In addition, the dose per administration can be, for example, 0.001 to 1000 µg or 0.01 to 100 µg in terms of the amount of the polynucleotide for antigen expression per 1 kg of the body weight of the subject.

The frequency of administration may be, for example, once, twice, three times, four times, or five times or more. In the case of multiple administrations, the interval of administration can be, for example, 1 to 2 weeks, 2 to 3 weeks, 3 to 4 weeks, 1 to 2 months, 2 to 3 months, or 3 to 6 months.

### (Combination)

The antigen composition, the composition for antigen expression, the antigen polypeptide or the polynucleotide for antigen expression can be used in combination with still another adjuvant. As the adjuvant, the adjuvant described above can be used.

### [Antibody composition]

The antibody composition of the present invention contains an antibody or antigen-binding fragment thereof having binding ability to at least one polypeptide selected from the group consisting of GPR17, CD34, GABRR1, OR2AG2, CMKLR1, CDH19, CD93, AVPR2, CCR7, and OXGR1 proteins.

### (Antibody, antigen-binding fragment)

In one embodiment, the antibody is an anti-GPR17 antibody, preferably an anti-GPR17 long isoform antibody, and more preferably an anti-human GPR17 long isoform antibody.

In one embodiment, the antibody can bind to at least one epitope included in a polypeptide consisting of an amino acid sequence at positions 1 to 36 of the human GPR17 long isoform. For example, the antibody can bind to a polypeptide including an amino acid sequence at positions 1 to 36 of the human GPR17 long isoform (SEQ ID NO: 5).

From the viewpoint of enhancing binding specificity to a photoaged cell, it is preferable that the antibody does not bind to the human GPR17 short isoform.

The sequences of the framework region and the constant region of the variable region of the antibody are not particularly limited as long as binding ability to the antigen is not lost, and for example, may be fragments of antibodies derived from a plurality of genes such as a humanized antibody, a human chimeric antibody, and a mouse chimeric antibody combined by a genetic engineering technique.

In the present specification, the "chimeric antibody" refers to an antibody obtained by combining a variable region derived from a non-human animal antibody and a constant region of a human antibody.

In the present specification, the "humanized antibody" refers to one obtained by transplanting a CDR (complementarity determining region) in a variable region derived from a non-human animal antibody into a human antibody, or one obtained by transplanting a CDR derived from a non-human animal antibody and a part of a framework region into a human antibody.

The isotype of the antibody of the present invention is not particularly limited, includes IgG (IgG1, IgG2, IgG3, IgG4), IgM, IgA (IgA1, IgA2), IgD, and IgE, and is preferably an IgG antibody.

The animal from which the antibody of the present invention is derived is not particularly limited, and examples thereof include rabbits, humans, mice, rats, sheep, goats, cows, horses, and guinea pigs, and among them, rabbits are preferable.

In the present specification, the "antigen-binding fragment" of an antibody refers to a partial fragment of an antibody capable of binding to an antigen, and includes, for example, Fab, Fab', F(ab')₂, Fv, ScFv, Sc(Fv)₂, a diabody, or a triabody. An overview of these antibody fragments is given in Hudson, P.J. et al., Nat. Med. 9 (2003) 129-134 and Plueckthun, A., In: The Pharmacology of Monoclonal Antibodies, Vol. 113, Rosenburg and Moore (eds.), Springer-Verlag, New York (1994), pp. 269-315.

The antibody or antigen-binding fragment thereof may be a drug conjugate in which one drug alone or two or more drugs are linked together depending on its use. Here, the link may be a covalent bond, or a coordinate bond, an ionic bond, a hydrophobic interaction, a van der Waals interaction, or other non-covalent bond.

Examples of the drug include cytotoxic agents, anti-aging components, antioxidant components, vitamins, anti-inflammatory agents, and cell activators.

The cytotoxic agent includes a chemotherapeutic agent, a radioisotope, a radiosensitizer or a photoresponsive probe [a phthalocyanine probe such as IRDye (registered trademark) 700DX (Nature Medicine, 2011, Vol. 17, pp. 1685-1691), malachite green, etc.], or the like. The linked body to which a cytotoxic agent is linked is suitably used for preventing or improving a photoaging state because the linked body can suppress or reduce an increase in photoaged cells by being delivered to a photoaged cell.

The anti-aging component, the antioxidant component, the vitamins, the anti-inflammatory agent, or the cell activator can improve the aging state itself of a photoaged cell or abnormality associated therewith, and thus are suitably used for preventing or improving the photoaging state.

A tag can be added to the antibody or antigen-binding fragment thereof of the present invention. Examples of such a tag include FLAG, HA (hemagglutinin), GST (glutathione-S-transferase), Myc, and polyhistidine (6×His tag, etc.). These tags can be used for immobilization, purification, detection, and the like of an antibody. An antibody or antigen-binding fragment to which the tag is added can be produced, for example, by preparing a product obtained by adding a polynucleotide encoding the tag to a polynucleotide encoding the antibody or antigen-binding fragment thereof of the present invention, and introducing the product into an in vivo protein expression system such as an animal cell or in vitro protein expression system such as a cell-free translation system.

The antibody can be obtained, for example, by performing the above-described method for inducing immunity on a subject for producing an antibody to separate an antibody secreted into plasma and purifying the antibody as appropriate. Alternatively, the antibody can be biosynthesized by preparing a hybridoma using an antibody-producing cell obtained from an immune-induced individual.

The antibody or antigen-binding fragment thereof can also be produced by a method in which a polynucleotide encoding these proteins is introduced into, for example, an animal cell (Chinese hamster ovary (CHO) cell, monkey kidney cell (COS), HEK 293 cell, NSO cell, HeLa cell, baby hamster kidney (BHK) cell, human hepatocellular carcinoma cell (for example, Hep G2), etc.), a non-animal eukaryotic cell (insect cell, etc.), a bacterium (E. coli, etc.), a yeast, an insect, a plant, a transgenic animal (cow, chicken, etc.), or an in vitro translation system (eukaryotic derived, E. coli-derived cell-free translation system, etc.) to express a target protein. In the production of a rabbit-derived antibody, a mouse chimeric antibody, a human chimeric antibody, a humanized antibody, or an antigen-binding fragment thereof, it is preferable to produce an antibody by such a polynucleotide introduction. The polynucleotide can be introduced using, for example, a plasmid, a cosmid, a bacteriophage, or the like as a vector. The introduction of the polynucleotide can be performed using a conventional genetic engineering technique.

The obtained antibody or antigen-binding fragment thereof may be appropriately fragmented (papain digestion, pepsin digestion, etc.), separated, purified, or the like. As the method of fragmentation, separation, and purification, known methods used for proteins and antibodies can be appropriately combined and used.

Furthermore, the method for producing the antibody or antigen-binding fragment thereof may include a step of linking various linking substances in order to prepare the linking body. These substances can be linked to any site on the antibody or antigen-binding fragment thereof, but from the viewpoint of not impairing the antigen-binding property, for example, a constant site is preferable. In the linking, an appropriate linker may be appropriately interposed.

### (Form of composition)

A specific embodiment of each of the antibody composition is not particularly limited, includes, for example, a pharmaceutical composition (including a pharmaceutical product or a quasi-pharmaceutical product), a cosmetic composition, a food (including a functional food such as a food for specified health uses, a food with functional claims, food with nutrient function claims or a supplement), or feed, and is preferably a pharmaceutical composition or a cosmetic composition.

The dosage form of the antibody composition may be appropriately selected depending on the intended use, and may be, for example, a dry product (lyophilized product, powder, etc.), a solution, a dispersion, a suspension, a tablet, a capsule, a troche, a liposome, a paste, a mousse, a gel, an emulsion, a cream, an aerosol, a spray, a sheet (carrying the substrate), or the like.

### (Other components of composition)

The antibody composition may optionally contain one or two or more additional components such as a carrier, an excipient, and a stabilizer depending on its use.

When the antibody composition is a pharmaceutical composition or a cosmetic composition, the antibody composition may contain, as those other components, the components exemplified in the above section [Antigen composition, composition for antigen expression] may be contained singly or in combination of two or more.

### (Function/use)

As also shown in the Examples, the antibody or antigen-binding fragment thereof can bind to the surface of a photoaged cell and also has binding ability to a live photoaged cell in a subject. Thus, when the antibody composition is administered to a subject, the contained antibody or antigen-binding fragment thereof binds to a photoaged cell. The immune response via the bound antibody or binding fragment thereof results in reduction or suppression of increase in photoaged cells, and thus can prevent or improve the photoaging state.

In addition, the antibody or binding fragment thereof can localize and deliver a linking substance linked thereto to a photoaged cell. Alternatively, for example, one or more antibodies or antigen-binding fragments can be associated with a drug delivery (DDS) carrier (for example, a vesicle) to deliver the DDS carrier containing the agent to a photoaged cell. Thereby, the antibody composition can selectively (preferably specifically) deliver the agent to a photoaged cell by being administered to a subject. Then, it is possible to reduce or suppress an increase in photoaged cells through the action of the agent, and thus prevent or improve the photoaging state.

According to the above, the antibody composition can be administered to a subject and used for delivering an agent to a photoaged cell in the subject.

In addition, the antibody composition can be administered to a subject in an effective amount thereof and used for reducing or suppressing an increase in photoaged cells in the subject.

Further, the antibody composition can be administered to a subject and used for preventing or improving a photoaging state in the subject.

Similarly, the antibody or antigen-binding fragment thereof can be used for producing a composition for (A) delivering an agent to a photoaged cell in a subject, (B) reducing or suppressing an increase in photoaged cells in a subject, or (C) preventing or improving a photoaging state in a subject.

Further, a composition containing the antibody or antigen-binding fragment thereof and a drug (preferably, the drug conjugate) can be administered to a subject and used for delivering an agent to a photoaged cell in a subject.

Furthermore, the antibody or antigen-binding fragment thereof can be administered to a subject in an effective amount thereof and used for preventing or improving a photoaging state in the subject.

The prevention or improvement of a photoaging state may be medical treatment or non-therapeutic.

### (Subject)

The subject to be administered is, for example, a mammal, includes a human or a non-human mammal (a non-human primate, a dog, a cat, a cow, a horse, a sheep, a pig, etc.), and is preferably a human.

The subject may be a person in need of prevention or improvement of a photoaging state, preferably, a person having a photoaging state or a person having a risk of a photoaging state.

### (Administration mode/dose)

Examples of the route of administration include percutaneous, intradermal, subcutaneous, transmucosal (nasal, sublingual, oral), intravascular, intraperitoneal, intrathecal, intramuscular, and oral, and the administration is preferably performed at a site where the photoaged cell is present.

In the administration, the dose per administration may be appropriately set according to the type of the antibody or binding fragment thereof to be used, the administration route, the attribute of the subject, and the like, and may be, for example, 0.0001 mg or more, 0.001 mg or more, or 0.01 mg or more, and 200 mg or less, 100 mg or less, 20 mg or less, 10 mg or less, 5 mg or less, 2 mg or less, 1 mg or less, 0.5 mg or less, 0.2 mg or less, or 0.1 mg or less per 1 kg of the body weight of the subject.

The frequency of administration may be, for example, once, twice, three times, four times, or five times or more. In the case of multiple administrations, the interval of administration can be, for example, 1 to 2 weeks, 2 to 3 weeks, 3 to 4 weeks, 1 to 2 months, 2 to 3 months, or 3 to 6 months.

### (Combination)

The antibody composition or the antibody or binding fragment thereof can be used in combination with still another agent (for example, another cytotoxic agent, anti-aging component, antioxidant component, vitamins, anti-inflammatory agent, cell activator, etc.).

### EXAMPLES

Hereinafter, the present invention will be described in more detail using Examples. However, these examples do not limit the present invention.

### [Test Example 1. Verification of method for preparing photoaged cell]

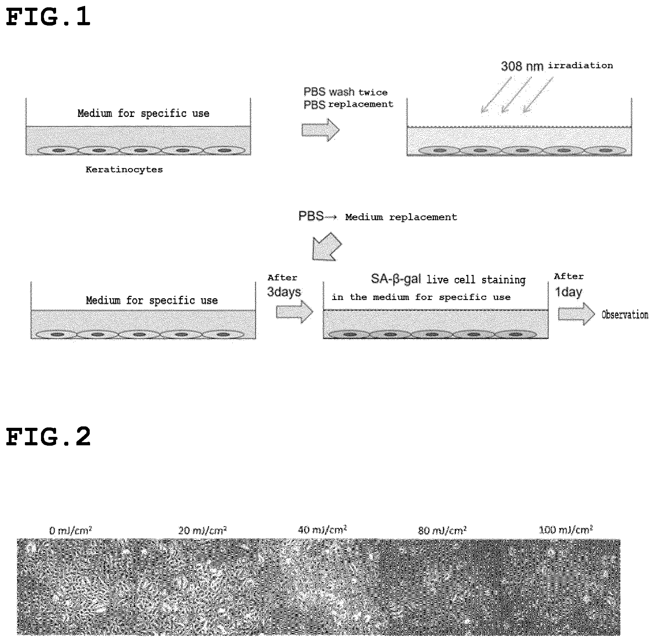

Normal human epidermal keratinocytes (NHEK), one type of human skin cells, were irradiated with the ultraviolet ray having a peak wavelength of 308 nm in the following procedure to prepare photoaged cells. Fig. 1 shows a schematic diagram of the procedure. Culture and incubation were performed at 37 °C using a CO₂ incubator. Cerabeam (registered trademark) UV 308 mini (manufactured by Ushio Inc.) was used as an ultraviolet light source. That is, the irradiated ultraviolet ray is light from a XeCl excimer discharge lamp as a light source that has passed through borosilicate glass (thickness: 1 mm) that cuts a wavelength around 280 nm, and its spectrum is shown in Figs. 8b and 9b of JP-A-2007-267936. The irradiated ultraviolet ray is UVB having no component in a region of less than 297 nm.
(1) NHEKs were cultured in a collagen coated dish (manufactured by Corning Incorporated) in a medium dedicated to keratinocytes (manufactured by LONZA) by a conventional method until the cells became sub-confluent.
(2) The medium was removed, the cells were washed twice with phosphate buffered saline (PBS), and then PBS was added so that the cells would be immersed adequately.
(3) The ultraviolet ray of 0 mJ/cm², 20 mJ/cm², 40 mJ/cm², 80 mJ/cm² or 100 mJ/cm² was irradiated. Each irradiation amount was obtained by setting the irradiation time to 0, 1, 3, 5, or 6 seconds.
(4) The PBS of the dish was replaced with a medium dedicated to keratinocytes (manufactured by LONZA KK.) and culture was performed for 3 days.
(5) The medium was removed by suction, 2 mL of SA-β-Gal pretreatment liquid (manufactured by Cell Biolabs, Inc.) was added to the medium, and incubation was performed for 2 hours.
(6) The SA-β-Gal pretreatment liquid was removed by suction, 10 µL of SA-β-Gal substrate solution (manufactured by Cell Biolabs, Inc.) was added to the medium, and culture was further performed for 1 day.

Fig. 2 is a diagram comparing microscopic images of photoaged cells prepared at each irradiation amount. As the irradiation amount increased, the number of cells decreased. However, detached cells (dead cells) were less observed. This indicates that when the irradiation amount was increased, the number of cells showing "cell division stop", which is a characteristic of aged cells, increased. That is, it was found that when the irradiation amount was 40 mJ/cm² or more, the number of cells that had stopped cell division remarkably increased, and when the irradiation amount was 80 mJ/cm² or more, aged cells that had sufficiently stopped cell division were obtained.

When the irradiation amount was increased, the size of cells increased. This represents "cell enlargement", which is a characteristic of aged cells.

All of the cells showing the cell division stop or the cell enlargement were stained with SA-β-Gal, and thus the cells were confirmed to be aged cells.

Thus, it was shown that living photoaged cells can be efficiently prepared by irradiation of the ultraviolet ray having a peak wavelength of 308 nm.

### [Test Example 2. Analysis of mRNA expression in photoaged keratinocytes]

Keratinocytes (UN group) with the irradiation amount of 0 mJ/cm² (without light irradiation) and keratinocytes (U80 group) irradiated with the ultraviolet ray at the irradiation amount of 80 mJ/cm² were prepared in the same procedure as in (1) to (5) of Test Example 1. Naturally aged human normal keratinocytes (P50 group) were also prepared by subculturing keratinocytes in a medium dedicated to keratinocytes for 50 days. Each group had n = 10. Cells of each group were collected from the dish, mRNA was extracted from the cells using TRIzol (registered trademark, manufactured by Thermo Fisher Scientific Inc.), and RNA-Seq analysis was performed. The read counts of all analyzed genes were normalized and compared between groups. The performance of RNA-Seq and the analysis of data were entrusted to DNA Chip Research Inc.

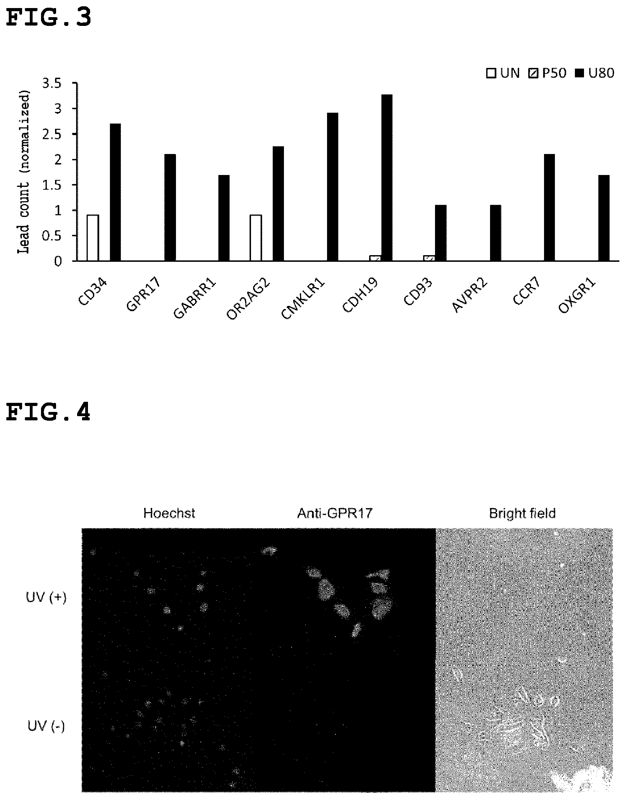

The comparison result of the read counts is shown in Fig. 3. It was found that GPR17, CD34, GABRR1, OR2AG2, CMKLR1, CDH19, CD93, AVPR2, CCR7, and OXGR1 were specifically increased in the photoaged cells prepared by ultraviolet irradiation.

From the above, it was shown that the 10 genes and the proteins thereof can be used as markers for detecting or quantifying photoaged cells.

Among them, mRNAexpression of GPR17, GABRR1, CMKLR1, AVPR2, CCR7, and OXGR1 was not observed except for the U80 group. These genes were shown to be markedly specific markers in photoaged cells.

### [Test Example 3. Preparation of anti-GPR17 monoclonal antibody]

An anti-GPR17 monoclonal antibody was isolated by the following method.
(1) An antigen peptide in which a cysteine residue was added to the C-terminus of an amino acid sequence at positions 1 to 36 of the human GPR17 long isoform (SEQ ID NO: 5) (GPR17LF peptide) was prepared by peptide synthesis. The peptide synthesis was outsourced to Cell Engineering Corporation. Keyhole limpet hemocyanin (KLH) or bovine serum albumin (BSA) as a carrier protein and MBS (m-maleimidobenzoyl-N-hydroxysulfosuccinimide ester) as a crosslinking agent were reacted with the cysteine residue at the C-terminus of the antigen peptide to bind thereto.
(2) The KLH-binding antigen peptide was administered to one rabbit (Sic: JW/CSK, 13 weeks old) in an amount of 2.5 mg at the start (after 0 day). Furthermore, as an additional immune antigen, a mixture obtained by mixing the same antigen peptide as that at the start with an adjuvant (TiterMax Gold; Funakoshi Co., Ltd.; Cat# CYT G-1) at a volume ratio of 1 : 1 was administered in an amount of 2.5 mg after 14 days, 28 days, and 42 days, respectively, for immunization. After 46 days, in order to confirm that the antibody titer was increased, blood was collected from the rabbit, and serum and lymphocytes were collected. The lymphocytes were cultured, and the culture supernatant was separated. The antibody titer of the obtained serum and culture supernatant was measured by ELISA using a plate on which the GPR17LF peptide was immobilized.
(3) Lymphocytes derived from rabbit spleen in which an increase in the antibody titer was confirmed were selected. To a single-cell ELISA plate on which the GPR17LF peptide was immobilized, lymphocytes diluted with RPMI medium (10% FBS) were added so that only one cell was contained in one well, and the plate was incubated for 45 minutes. Positive clones were identified by detecting an anti-GPR17 antibody bound to the antigen of the plate with a labeled anti-rabbit IgG antibody. As a result, 2×10⁵ lymphocytes were screened to obtain 32 positive clones. From the obtained positive clones, IgG heavy chain and light chain genes of each cell were amplified using a PCR method.
(4) A CAG promoter was added to the obtained heavy chain and light chain genes of each cell using a PCR method. The resulting polynucleotide containing the heavy chain and light chain genes was introduced into HEK293 cells and cultured in RPMI medium for 3 days. Then, the culture supernatant containing a monoclonal antibody was collected.
(5) The anti-GPR17 antibody titer in the culture medium supernatant of each of the obtained clones was measured by ELISA. An ELISA plate coated with GPR17LF peptide was used as a plate, and an anti-rabbit IgG antibody was used as a secondary antibody.

As a result, 15 clones of monoclonal antibodies having binding ability to the GPR17LF peptide were obtained. Therefore, it was shown that immunity of a subject is induced by a peptide derived from GPR17, and an anti-GPR17 antibody is reproducibly obtained by antibody screening.

### [Test Example 4. Detection of photoaged cell using anti-human GPR17 monoclonal antibody]

### (Preparation of photoaged cell)

Cells irradiated with ultraviolet rays were prepared in the same manner as in Test Example 1 except that a collagen-coated glass plate was used as a cell culture substrate. The irradiation amount was 0 mJ/cm² (UV(-) group) or 80 mJ/cm² (UV(+) group). UV irradiation was performed for 5 seconds. As the collagen-coated glass plate, one obtained by adding 20 µL/well of 0.1 mL of Cell Matrix Type I-C (manufactured by Nitta Gelatin Inc.) added to 0.9 mL of a diluent (dilute hydrochloric acid pH 3.0) to Multitest slide, 8-well (manufactured by MP BIOMEDICALS), allowing the mixture to stand at room temperature for 30 minutes, and then washing the mixture twice with 20 µL/well of a keratinocyte dedicated medium was used.

Next, the obtained cultured cells were stained and observed by the following procedure.
(1) The medium was removed by aspiration, 0.1 mL of a solution containing about 1 µg/mL of one clone of the anti-human GPR17 monoclonal antibody obtained in Test Example 3 was added as a primary antibody, and the mixture was incubated at 4°C for 1 hour. Hoechst was used for nuclear staining.
(2) Cells were washed three times on ice with 0.2% (w/v) BSA-containing phosphate buffered saline (PBS).
(3) Anti-rabbit IgG Cy3 conjugate (manufactured by Jacson ImmunoReserch Laboratry Inc.) diluted 500 times with PBS containing 0.2% (w/v) BSA was added as a secondary antibody, and the mixture was incubated at 4°C for 30 minutes.
(4) Cells were washed on ice three times with PBS containing 0.2% (w/v) BSA and one more time with PBS.
(5) Keratinocytes obtained by SlowFade (trademark) Gold antifade reagent (manufactured by Thermo Fisher Scientific Inc.) were embedded on a glass slide, and observed with a fluorescence microscope.

The obtained stained images are shown in Fig. 4. Cells in the UV(+) group were stained with anti-human GPR17 monoclonal antibody, while cells in the UV(-) group were not stained. From the above, it was shown that the obtained anti-GPR17 monoclonal antibody had binding ability to a photoaged cell.

## Claims

1. An antigen composition for inducing immunity against a photoaged cell in a subject, comprising
at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide,
in which the at least one polypeptide is bound to a carrier protein or is not bound to a carrier protein.

2. An antigen composition for reducing or suppressing an increase in photoaged cells of a subject, comprising
at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide,
in which the at least one polypeptide is bound to a carrier protein or is not bound to a carrier protein.

3. The antigen composition according to claim 1 or 2, comprising an immunogenic GPR17 polypeptide.

4. The antigen composition according to claims 1 or 2, in which the immunogenic GPR17 polypeptide includes 3 to 40 consecutive amino acid residues in a region selected from the group consisting of positions 1 to 51, positions 123 to 127, positions 198 to 212 and positions 293 to 295 of GPR17 long isoform.

5. The antigen composition according to claim 1 or 2, in which the immunogenic GPR17 polypeptide includes 3 to 40 consecutive amino acid residues in the region at positions 1 to 51 of the GPR17 long isoform.

6. The antigen composition according to claim 1 or 2, in which the immunogenic GPR17 polypeptide includes a polypeptide consisting of an amino acid sequence of SEQ ID NO: 5.

7. The antigen composition according to claim 1 or 2, in which the at least one polypeptide has 80% or more identity to a corresponding wild-type polypeptide.

8. The antigen composition according to claim 1 or 2, further comprising an adjuvant.

9. The antigen composition according to claim 1 or 2, for preventing or improving a photoaging state.

10. A composition for antigen expression for stimulating immunity against a photoaged cell in a subject, comprising a polynucleotide encoding at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide.

11. A composition for antigen expression for reducing or suppressing an increase in photoaged cells of a subject, comprising a polynucleotide encoding at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide.

12. The composition for antigen expression according to claim 10 or 11, comprising a polynucleotide encoding an immunogenic GPR17 polypeptide.

13. The composition for antigen expression according to claim 10 or 11, in which the immunogenic GPR17 polypeptide includes 3 to 40 consecutive amino acid residues in a region selected from the group consisting of positions 1 to 51, positions 123 to 127, positions 198 to 212 and positions 293 to 295 of GPR17 long isoform.

14. The composition for antigen expression according to claims 10 or 11, in which the immunogenic GPR17 polypeptide includes 3 to 40 consecutive amino acid residues in the region at positions 1 to 51 of the GPR17 long isoform.

15. The composition for antigen expression according to claims 10 or 11, in which the immunogenic GPR17 polypeptide includes a polypeptide consisting of an amino acid sequence of SEQ ID NO: 5.

16. The composition for antigen expression according to claims 10 or 11, in which the at least one polypeptide has 80% or more identity to a corresponding wild-type polypeptide.

17. The composition for antigen expression according to claims 10 to 16, further comprising an adjuvant.

18. The composition for antigen expression according to claims 10 or 11, for preventing or improving a photoaging state.

19. A method for inducing immunity against a photoaged cell, comprising administering to a subject:
(i) at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide,
the polypeptides being linked or not being linked to a carrier protein;
(ii) a polynucleotide encoding at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide; or
(iii) a combination of (i) and (ii).

20. A method for reducing or suppressing an increase in photoaged cells of a subject, comprising administering to a subject in need thereof an effective amount of:
(i) at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide,
the polypeptides being linked or not being linked to a carrier protein;
(ii) a polynucleotide encoding at least one polypeptide selected from the group consisting of an immunogenic GPR17 polypeptide, an immunogenic CD34 polypeptide, an immunogenic GABRR1 polypeptide, an immunogenic OR2AG2 polypeptide, an immunogenic CMKLR1 polypeptide, an immunogenic CDH19 polypeptide, an immunogenic CD93 polypeptide, an immunogenic AVPR2 polypeptide, an immunogenic CCR7 polypeptide, and an immunogenic OXGR1 polypeptide; or
(iii) a combination of (i) and (ii).

21. An antibody composition for reducing or suppressing an increase in photoaged cells of a subject, comprising an antibody or antigen-binding fragment thereof having binding ability to at least one polypeptide selected from the group consisting of GPR17, CD34, GABRR1, OR2AG2, CMKLR1, CDH19, CD93, AVPR2, CCR7, and OXGR1 proteins.

22. An antibody composition for delivering a drug to a photoaged cell of a subject, comprising an antibody or antigen-binding fragment thereof having binding ability to at least one polypeptide selected from the group consisting of GPR17, CD34, GABRR1, OR2AG2, CMKLR1, CDH19, CD93, AVPR2, CCR7, and OXGR1 proteins.

23. The antibody composition according to claim 21 or 22, comprising an anti-GPR17 antibody or antigen-binding fragment thereof.

24. A method for delivering a drug to a photoaged cell in a subject, comprising administering to a subject in need thereof:
a composition containing an antibody or antigen-binding fragment thereof having binding ability to at least one polypeptide selected from the group consisting of GPR17, CD34, GABRR1, OR2AG2, CMKLR1, CDH19, CD93, AVPR2, CCR7, and OXGR1 proteins, and a drug.

25. A method for reducing or suppressing an increase in photoaged cells of a subject, comprising administering to a subject in need thereof an effective amount of an antibody or antigen-binding fragment thereof having binding ability to at least one polypeptide selected from the group consisting of GPR17, CD34, GABRR1, OR2AG2, CMKLR1, CDH19, CD93, AVPR2, CCR7, and OXGR1 proteins.
